# FASCICULE DE BREVET EUROPEEN

(11) **EP 4 551 415 B1**
(45) Date de publication et mention de la délivrance du brevet: **05.11.2025**
(21) Numéro de dépôt: 23736189.4
(22) Date de dépôt: 14.06.2023
(51) Int. Cl.: B60H 1/24, B60H 3/06, B60H 3/00, B01D 53/04, B60H 1/34, F24F 8/10, F24F 8/108, F24F 8/158, F24F 8/192, F24F 8/20, B01D 46/54, A61L 9/16

(54) **SYSTEME DE FILTRATION D'AIR ET DE DIFFUSION D'AIR PURIFIE DANS UN HABITACLE DE VEHICULE**
SYSTEM ZUM FILTERN VON LUFT UND ZUM VERTEILEN GEREINIGTER LUFT IN EINEN FAHRZEUGINNENRAUM
SYSTEM FOR FILTERING AIR AND FOR DIFFUSING PURIFIED AIR INTO A VEHICLE INTERIOR

(30) Priorité: 08.07.2022 FR 2207062
(43) Date de publication de la demande: 14.05.2025
(73) Titulaire: Purflux Filtration, 78280 Guyancourt (FR)
(72) Inventeur: LE BLOA, Laurent, 14760 BRETTEVILLE-SUR-ODON (FR)
(74) Mandataire: Plasseraud IP
(86) Numéro de dépôt international: PCT/FR2023/050869
(87) Numéro de publication internationale: WO 2024/009019

(56) Documents cités:
- CN-A- 107 158 858
- CN-U- 208 771 066
- JP-A- 2005 343 425
- US-A- 5 167 129
- US-A1- 2007 017 194
- US-A1- 2019 160 416

## Description

### Domaine technique

L'invention concerne les équipements de filtration d'air dans des zones abritées des conditions extérieures météorologiques, en particulier les systèmes de filtration de l'air d'habitacles, par exemple pour permettre de purifier l'air d'un habitacle d'un véhicule, notamment de véhicule motorisé pendant, avant ou après son fonctionnement.

### Arrière-plan technologique

Dans les applications de filtration d'air habitacle, il est connu d'utiliser des éléments filtrants pour filtrer les flux de fluide ou les fluides gazeux, par exemple pour filtrer un flux d'air qui est fourni à l'intérieur de la cabine d'un véhicule automobile. L'élément filtrant est intégré dans un circuit d'alimentation en air provenant de l'extérieur, avec des fonctions de ventilation et/ou climatisation. Dans le domaine automobile, l'équipement de ventilation et contrôle de température est couramment désigné HVAC (de l'anglais « Heating, Ventilation and Air-Conditioning »). Ce système est formé à l'avant du véhicule et associé à des commandes accessibles par le conducteur.

L'augmentation de la pollution atmosphérique, en particulier dans les grandes villes, associée à l'utilisation de tels équipements de climatisation dans les véhicules, provoque une altération très importante de la qualité de l'air présente/admise dans l'habitacle du véhicule si l'air n'est pas purifié et traité, au moyen des filtres de cet équipement HVAC.

Afin d'éviter l'altération de la qualité d'air dans l'habitacle, des systèmes prévoient la recirculation de l'air intérieur, via une boucle intégrée à l'équipement HVAC. Cependant, ces systèmes peinent à purifier efficacement l'air. En outre, les passagers ont rapidement une sensation de désagrément/ mal des transports lorsque l'équipement HVAC renvoie de l'air en boucle.

Il a été aussi proposé d'intégrer, en tant que fonctionnalité supplémentaire de l'équipement de climatisation, une régénération du filtre à air, comme dans le document FR 2976856 par exemple. Cependant, cela complexifie grandement la conception de l'équipement, ainsi que son pilotage.

Le document US5167129 décrit un système de purification d'air pour un habitacle automobile. Le système comprend un agencement filtrant incluant un média filtrant et une chambre de filtration dans laquelle s'étend le média filtrant. Il comprend également une ou plusieurs voies d'admission, un ventilateur pour faire circuler l'air provenant de l'habitacle, et une conduite d'évacuation d'air purifié. La conduite d'évacuation est en communication avec deux branches et des moyens de diffusion d'air purifié longeant le toit de l'habitacle afin de diffuser l'air purifié de manière descendante dans l'habitacle. Ces moyens présentent des perforations et/ou des zones perméables à l'air. Les documents US2019160416 et JP2005343425 décrivent des dispositifs similaires.

CN208771066U présente un filtre à particules ultrafines capable de filtrer les matières particulaires PM0.1 avec une efficacité dépassant 99 %. De plus, CN107158858A introduit un filtre électrostatique doté d'un pré-filtre et d'une couche conçue pour capturer les composés volatils. Cette couche de filtration est efficace pour filtrer les particules PM0.3, PM2.5 et PM10, ainsi que les polluants aériens tels que le pollen et les allergènes.

Par ailleurs, les systèmes existants montrent une efficacité très limitée pour éviter de faire recirculer des agents pathogènes comme les virus. Plus généralement, au regard du risque d'inhalation de particules fines pouvant s'avérer néfastes, il existe un besoin pour mieux garantir un air habitacle sain.

### Résumé

Afin d'améliorer la situation, il est proposé selon l'invention un système de purification de l'air d'un habitacle suivant la revendication 1.

La présence d'un agencement ou élément filtrant de type HEPA (pour la filtration de particules aériennes à haute efficacité), qui présente un média filtrant adapté, par exemple avec au moins une couche de membrane ePTFE intégrée, pour remplir les critères de la classe de filtre H13, offre un avantage pour éviter de faire recirculer des particules fines indésirables dans l'habitacle. Le recours à des gaines, par exemple à des gaines textiles (structures microperforées dans des options), permet à la fois une adaptation à une géométrie de toit de l'habitacle en ayant l'avantage de pouvoir :
- dé-stratifier (en particulier dans des périodes de froid/en hiver où les apports d'un chauffage sont faibles dans le bas de l'habitacle, du fait de la montée d'air chaud) ; et
- de climatiser tout le volume de l'habitacle intérieur lorsque le véhicule est soumis à des conditions de chaleur importante (typiquement en été).

Selon une particularité, les moyens de diffusion d'air purifié délimitent des passages de circulation d'air périphériques par rapport à une zone d'occupation de passagers dans l'habitacle, par exemple en étant accolés à une cloison externe de véhicule. Plus généralement, les passages de circulation peuvent inclure au moins un passage unidirectionnel et de section sensiblement constante, dans lequel le débit décroît en s'éloignant de la chambre de filtration et/ou au en allant vers l'avant du véhicule, en raison de la diffusion d'air purifié au travers des parois de gaine délimitant ce tronçon, la diffusion étant permise du côté du volume interne de l'habitacle.

Dans des formes de réalisation du système selon l'invention, on peut avoir recours à une ou plusieurs des caractéristiques suivantes :
- la chambre de filtration, la conduite d'évacuation et les passages périphériques de circulation d'air (inclus au moins pour partie dans les moyens de diffusion) font partie d'un même circuit de circulation d'air.
- ce circuit de circulation d'air est indépendant et séparé d'un équipement de climatisation et contrôle de température par filtration et conditionnement d'un air prélevé à l'extérieur de l'habitacle.
- le moyen de mise en circulation d'air est configuré pour être placé dans une région arrière de l'habitacle (par référence à un sens de circulation routière dit vers l'avant du véhicule définissant l'habitacle).
- le système est autonome, en étant indépendant de l'équipement à l'avant formant l'équipement ou partie de climatisation (équipement de ventilation et contrôle de température couramment désigné HVAC).
- les perforations et/ou zones perméables à l'air, de préférence pour chaque gaine, sont intégrées dans une épaisseur de matériau de gaine.
- les moyens diffuseurs consistent une partie entièrement fixe perméable aux gaz, passive et située à l'extrémité supérieure du système, sachant que la circulation d'air est actionnée à distance, ailleurs que dans les moyens diffuseurs.
- la circulation d'air depuis le moyen de mise en circulation d'air, tel qu'un ventilateur par exemple, qui se situe dans une partie de base du système verticalement espacée des moyens diffuseurs.
- les perforations et/ou zones perméables à l'air s'étendent entièrement dans l'épaisseur de matériau (par exemple textile) de la gaine, et/ou s'étendent sans former de reliefs saillants sur la surface externe de la gaine.
- les gaines peuvent être coudées et/ou présenter une forme de « T », sur au moins un côté de l'habitacle (droit ou gauche).
- les gaines peuvent recouvrir par l'intérieur une colonne arrière (colonne séparant de préférence deux zones à vitrage dont l'une se situe sur une porte) du véhicule, dans un tronçon ascendant des moyens de diffusion qui est relié à la conduite d'évacuation.
- seule l'extrémité supérieure de la colonne arrière peut être recouverte par un tronçon de la gaine qui sert : pour une circulation ascendante de l'air purifié, par un passage principal ; et pour diffuser une fraction de l'air purifié (ascendant), en ayant un effet de réduction du débit ascendant dans un canal ou passage unidirectionnel délimité par le tronçon considéré.
- lorsqu'il est prévu des gaines, tout ou partie de celles-ci sont des gaines souples (en matériau déformable, par exemple élastiquement déformable et à mémoire de forme).
- les moyens de diffusion d'air purifié comprennent sélectivement des gaines souples.
- la plus grande dimension en section transversale d'une ou plusieurs des gaines souples (éventuellement de toutes ces gaines) peut optionnellement être inférieure ou égale à 10 ou 15 mm.
- les gaines incluent une matière textile définissant un revêtement extérieur de la gaine, visible depuis l'intérieur de l'habitacle ou recouvert par une couche protectrice qui est optionnellement plus rigide que la gaine.

Selon une particularité, l'une au moins des gaines est une gaine textile qui a une forme, en coupe transversale, choisie parmi :
- la forme demi-circulaire ;
- la forme en quart de rond ;
- le forme segmentaire (de moindre section que la forme demi-circulaire) ;
- la forme sectorielle (qui peut être avantageuse pour s'adapter à une zone latérale de plafond formant un angle différent de 90°, par exemple supérieure à 90°).
Dans des exemple d'intégration, une telle gaine peut être accolée à une surface sensiblement plane correspondant au toit du véhicule.

Dans des modes de réalisation, le système présente un support qui est commun : aux moyens de diffusion, prévus pour diffuser une fraction de l'air purifié ascendant ; et à des organes de diffusion de produits de confort olfactif (fragrance par exemple) et/ou d'hygiène, en particulier biocide. Eventuellement, de tels organes sont agencés pour traverser l'épaisseur des moyens de diffusion.

Dans des formes de réalisation de la partie de traitement d'air du système selon l'invention, on peut avoir recours à une ou plusieurs des caractéristiques suivantes :
- la partie de traitement d'air comprend un boîtier qui délimite la chambre de filtration et est pourvu d'une entrée de boîtier formant tout ou partie de la ou des voies d'admission, pour faire circuler de l'air (admis dans la ou les voies d'admission) dans la zone d'amont de la chambre de filtration.
- la zone d'aval est séparée de la zone amont en étant délimitée par un élément filtrant préassemblé incluant le média filtrant purificateur d'air.
- la zone d'aval est en communication avec la conduite d'évacuation par l'intermédiaire d'une zone basse de ventilation, verticalement distale du toit.
- le média filtrant purificateur d'air est du type panneau ou éventuellement à extension annulaire autour d'un axe central.
- le média filtrant est optionnellement conçu pour permettre une filtration d'air centripète.
- la couche pour la captation de composés volatils est agencée intérieurement par rapport au média filtrant ou tout du moins en aval de cette dernière.
- la couche pour la captation de composés volatils s'étend autour d'un espace creux, l'espace creux formant de préférence une partie de la zone d'aval de la chambre de filtration.
- la partie de traitement d'air est située au même niveau ou plus bas qu'une partie d'assise prévue dans l'habitacle automobile (dans une zone basse, en particulier sous le niveau des vitres du véhicule).

Dans des options de réalisation, on prévoit une ou plusieurs des particularités suivantes pour le préfiltre :
- le préfiltre est configuré pour séparer des particules de diamètre ou taille caractéristique submillimétrique.
- le préfiltre est disposé au niveau d'une entrée d'admission du système qui présente au moins une fente allongée ou des ouvertures distribuées sur une largeur de l'habitacle.
- l'entrée d'admission est située sous une partie d'assise des sièges arrière et/ou entièrement sous le niveau d'ouvertures de fenêtre obturées par des vitres (zone de vitrage du véhicule).

Selon une particularité, la zone basse de ventilation est équipée d'au moins un élément ventilateur qui constitue tout ou partie du moyen de mise en circulation d'air. En variante, une pompe à air peut être prévue dans la zone basse pour constituer tout ou partie du moyen de mise en circulation d'air.

Dans des options de réalisation des gaines permettant de former les zones de diffusion pour le retour d'air (purifié) dans l'habitacle, il est prévu deux tronçons de gaine textile qui sont par exemple répartis sur deux côtés ou flancs opposés dans l'habitacle. Il peut être prévu pour ces tronçons une ou plusieurs des particularités suivantes :
- les deux tronçons sont continus, éventuellement avec une sous-partie sensiblement rectiligne suivant une direction d'arrière-en-avant.
- les deux tronçons, continus, forment tout ou partie de l'au moins une gaine.
- les deux tronçons s'étendent chacun continûment de l'extrémité arrière du toit jusqu'à l'extrémité avant du toit, par exemple en définissant chacun une section utile de diffusion d'air purifié au moins égale à 4 dm², par exemple supérieure ou égale à 6 dm². Un total de section utile d'environ 0,5 m² peut être optionnellement prévu.
- les tronçons sont conçus avec une structure textile, permettant de délimiter un canal/passage principal de circulation d'air purifié qui se vide au fur-et-à mesure qu'on avance suivant la direction d'écoulement.
- les tronçons de gaine textile sont agencés sans zone particulière d'accélération d'air et/ou configurées pour permettre une diffusion uniforme, en particulier sans courant d'air.
- les tronçons de gaine textile sont agencés pour vider suffisamment les passages de circulation, de façon à autoriser des débits d'air significatif, par exemple avec un débit supérieur ou égal à 10 m³/h (par exemple de l'ordre 20 ou 30 m³/h) pour un premier paramétrage de commande déterminé du moyen de mise en circulation d'air, ce dernier étant adapté pour fonctionner avec au moins un deuxième paramétrage correspondant à un débit plus bas, par exemple inférieur ou égal à 2 m³/h. Avec un filtre HEPA H13, un renouvellement d'air 5 fois par heure peut être prévu (ce qui peut permettre de définir le débit volumique en fonction du volume de l'habitacle).
- les tronçons peuvent présenter chacun une face inférieure permettant de diffuser de l'air purifié directement vers le bas.
- les deux tronçons sont répartis sur des côtés opposés de l'habitacle en présentant des faces en regard l'une de l'autre pour diffuser aussi de l'air purifié suivant une direction parallèle au toit du véhicule.

Eventuellement, le système de purification est configuré pour se commander à distance, par une télécommande dédiée ou par un terminal de communication tel qu'un téléphone (smartphone) disposant d'une application (dédiée ou plus générale), de sorte qu'une interface homme-machine soit formée par cet outil (télécommande ou terminal de communication). Au moins un paramètre représentatif d'un débit de circulation dans les moyens de diffusion peut ainsi être ajusté. Cette disposition peut permettre, le cas échéant de commander une phase de purification en avance de l'occupation de l'habitacle par des occupants, par exemple 30 minutes ou 1 heure avant un début de conduite envisagée pour le véhicule V équipé du système 1. Dans un tel cas, on comprend que le système autonome peut être actif, typiquement sans déclencher l'équipement HVAC prévu pour insuffler de l'air extérieur dans l'habitacle.

Dans des options de diffusion d'air purifié grâce au système, on peut utiliser une large variété de structure perméable, de taille de perforations et/ou de pores dans la partie à l'interface avec l'habitacle intérieur, le cas échéant avec différentes tailles de microperforations dans un même tronçon de gaine ou tronçon des moyens de diffusion.

Optionnellement, les moyens de diffusion d'air purifié disposent d'un ou plusieurs organes de diffusion pour la diffusion d'au moins un produit volatile, sec, par exemple choisi parmi un composant biocide et une fragrance parfumée. Un réservoir commun peut être prévu pour un produit ou une composition donnée (qui peut être un mélange combinant une flagrance et un produit biocide), le cas échéant avec des liaisons divergentes pour une distribution vers des organes espacés/répartis dans une zone supérieure de l'habitacle et/ou contre le toit du véhicule. Typiquement, chacun des organes de diffusion peut être en connexion fluidique avec un réservoir correspondant de produit ; les organes de diffusion sont de préférence supportés par ou rendus solidaires des gaines ou éléments structurels des moyens de diffusion d'air purifié.

Selon un autre aspect, il est proposé une utilisation combinée d'un agencement filtrant et de moyens de diffusion d'air purifié dans un habitacle pour passager et/ou conducteur, dans un véhicule, l'agencement filtrant et les moyens de diffusion d'air purifié étant interconnectés à l'arrière du véhicule pour former le système de purification de l'air tel que défini plus haut, ce grâce à quoi l'air purifié diffusé au travers de gaines des moyens de diffusion d'air, dans différentes positions adjacentes au toit du véhicule, par exemple suivant la direction d'avant en arrière du véhicule, est exempt de particules fines PM0,3 et PM0,1.

Grâce à ce type de purification, l'habitacle peut être débarrassé des principales impuretés s'accumulant en partie basse de l'habitacle et/ou à l'arrière de l'habitacle (par défaut d'aération active) et les virus ou agents pathogène similaires peuvent être efficacement éliminés.

Avantageusement, l'air d'habitacle peut être capté à un niveau inférieur à celui du niveau de diffusion d'air purifié, l'agencement de filtration pouvant être efficacement alimenté pour une séparation des particules et composants nocifs, indépendamment d'un apport/renouvellement par de l'air extérieur. Cela permet de cibler la pollution intérieure dans un véhicule et le recours à une partie de traitement qui a un niveau de séparation élevé, en pratique de biveau H13, évite des contaminations des passagers par inhalation de particules ou matières nocives qui ont tendance à stagner naturellement en bas de l'habitacle mais qui peuvent être soulevées/brassées avec l'air intérieur en l'absence d'un tel système purificateur.

Selon une option, la totalité des composants de filtration et des composants actifs pour l'épuration et la mise en circulation de l'air du système sont situés dans une partie arrière du véhicule en permettant un fonctionnement du système indépendant du fonctionnement d'un équipement de climatisation intégré à l'avant du véhicule qui est configuré pour réaliser la climatisation et le contrôle de température par filtration et conditionnement d'un air prélevé à l'extérieur de l'habitacle.

Dans des réalisations, les moyens de diffusion d'air purifié comprennent au moins deux zones de diffusion séparées l'une de l'autre par un dispositif d'aiguillage présentant au moins une vanne réglable ou un clapet, afin d'ajuster un débit de diffusion d'air sélectivement en fonction des zones de diffusion, avec de préférence une des deux zones de diffusion agencée pour former une zone avant de diffusion, par exemple associée à un premier rang d'assise du véhicule et une autre des deux zones de diffusion agencée pour former une zone arrière de diffusion, par exemple associée à un deuxième rang d'assise du véhicule.

Avec cette disposition, on prévoit un agencement de filtration d'air extérieur de l'équipement HVAC, qui peut fonctionner par exemple indépendamment du système HVAC, de façon autonome si une source d'alimentation est prévue dans le système (batterie électrique ou raccord pour alimentation électrique). En outre, un tel agencement est typiquement compatible avec une opération de rechange, aisée, de tout ou partie des composants participant à la filtration.

Il est par ailleurs permis d'utiliser un ou des composants d'amélioration du confort olfactif, libérés par diffusion lente ou par intermittence.

Dans certaines options, l'élément/agencement filtrant peut être en partie ou entièrement inséré dans un tronçon de conduite ou dans un boîtier qui, éventuellement, peut se changer avec l'élément filtrant si ce dernier n'est pas monté détachable par rapport à cet élément périphérique non filtrant. De préférence cependant, la rechange ne concerne que l'élément/agencement filtrant en tant que tel, ce qui permet par exemple de réutiliser la conduite de circulation d'air dans lequel l'élément filtrant délimite les zones amont et aval.

Dans des formes de réalisation, le système est activé à distance par un utilisateur, alors que le véhicule est stationnement, en prévision d'un trajet. Un mode de purification à débit maximal peut ainsi être paramétré, le cas échéant. Ce mode à haut débit peut ensuite être optionnellement coupé lorsque le véhicule démarre et roule. Le cas échéant, le système peut être éteint lorsque le véhicule roule et que l'équipement de type HVAC est en fonctionnement.

### Brève description des dessins

D'autres caractéristiques, détails et avantages apparaîtront à la lecture de la description détaillée ci-après, et à l'analyse des dessins annexés, sur lesquels :
La figure 1 est un schéma montrant un habitacle intérieur et illustrant un exemple de mise en place d'une circulation d'air avec effet de purification au niveau d'un partie de traitement incluant des couches de filtration, par un système conforme à l'invention.
La figure 2 est une vue de dessus schématique d'un système tel que celui montré sur la figure 1, intégré dans un habitacle intérieur.
La figure 3 est une vue en perspective d'une partie de traitement permettant la purification et la mise en circulation d'air prélevé en bas de l'habitacle.
La figure 4 montre un exemple d'élément filtrant, combinant des couches de filtration dont l'une correspond à une filtration de niveau H13, et permettant une filtration centripète.
La figure 5 illustre un boîtier de filtration permettant d'installer plusieurs éléments filtrant formant des couches de filtration agencées en série, dont l'une permet une filtration de niveau H13.
La figure 6 illustre un agencement optionnel d'une gaine formant partie des moyens de diffusion d'air purifié, ici avec trois régions de diffusion présentant des différences de surface correspondant à plusieurs modes de diffusion douce d'air.
La figure 7 montre un exemple d'agencement filtrant combinant trois couches différentes dans un seul bloc.

### Description de modes de réalisation

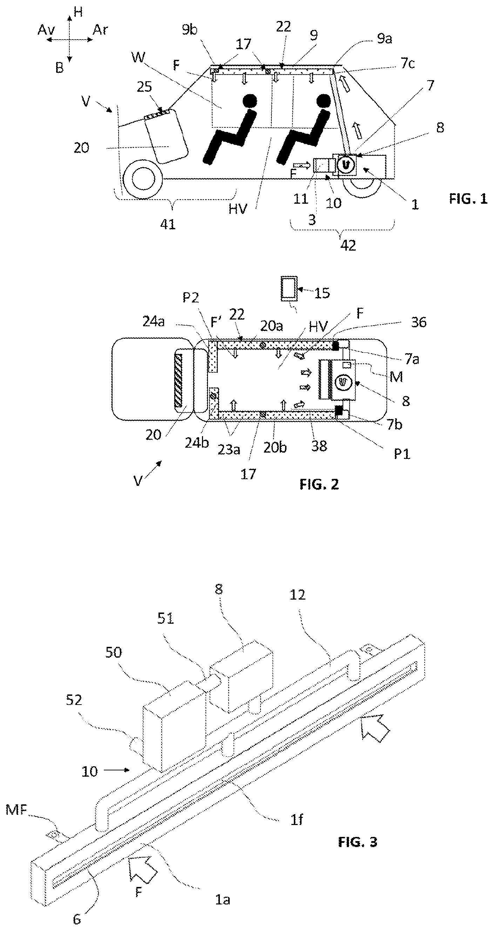

En référence aux figures 1 et 3, on peut voir schématiquement l'intérieur d'un habitacle HV de véhicule V, dans lequel de l'air peut être acheminé de l'extérieur par un équipement 20 de climatisation de type HVAC. Cet air extérieur peut être épuré par le filtre à air 25 prévu dans cet équipement 20 installé à l'avant du véhicule V. Lorsque les portes et fenêtres W du véhicule V sont maintenues fermées, les impuretés contenues dans l'habitacle intérieur HV risque de s'accumuler et l'air intérieur devient de plus en plus pollué.

Il est prévu un système 1 de purification par filtration de cet air intérieur, pourvu d'un agencement filtrant 2 qui intègre au moins un média filtrant 3 ayant un niveau H13 (classe de filtration H13) pour la filtration de particules fines. Un tel média 3, de classe H13 selon la norme ISO 29463 ou EN 1822 ou mieux (remplissant au moins les critères de la classe H13), participe à assainir l'air intérieur F' admis via une ou plusieurs voies d'admission 6.

Ce média 3 peut être complété par une couche 4 additionnelle de filtration, par exemple pour la captation de composés volatils, en particulier par adsorption moléculaire. Dans une option non limitative la couche 4 inclut du charbon actif déposé/supporté sur un substrat perméable à l'air. Le média filtrant 3 et la couche 4 peuvent être éventuellement logés dans une même chambre de filtration 5 qui communique avec une conduite d'évacuation 7.

En référence aux figures 1, 3 et 5, le système 1 peut inclure une partie de traitement 10 assurant la purification de l'air prélevé dans l'habitacle intérieur HV. Dans cette partie de traitement 10, le média filtrant 3 peut être conformé selon différentes géométries : à plat, plissé, conformé de façon annulaire. Le média filtrant 3 s'étend à l'intérieur d'un boîtier 50 dans lequel on forme un agencement ou élément filtrant 2, apte à séparer une zone d'amont Z1 d'une zone d'aval Z2 dans la chambre de filtration 5 délimitée par le boîtier 50. L'air à purifier F, issu de l'habitacle HV, est acheminé dans la chambre 5 par une entrée 51 du boîtier 50 communiquant avec la zone d'amont Z1. Un préfiltre 11 est prévu, soit dans la zone d'amont Z1, à l'intérieur du boîtier 50, soit au niveau de l'entrée 51, soit encore dans une conduite ou voie d'admission 6 formant une entrée commune du système 1.

Le média 3, éventuellement pourvu de plis, peut être multicouches. L'un au moins parmi un préfiltre 11 et le média filtrant 3 peut être fibreux, à base de coton, cellulose, de fibres synthétiques par exemple ou d'un mélange de fibres.

Optionnellement, le préfiltre 11 peut inclure ou consister en une grille, par exemple en formant une couche ou élément barrière aux particules solides de taille submillimétrique. Plus généralement, le préfiltre 11 est apte à retenir les particules solides ayant une dimension supérieure ou égale à 0,1 mm. Dans l'exemple de la figure 3, une fente 1f ou plusieurs fentes peuvent être disposées dans une région inférieure de l'habitacle pour permettre une admission d'air, avec un effet d'aspiration généré par un ventilateur ou autre moyen 8 similaire de mise en circulation d'air, placé plus en aval que le préfiltre 11 suivant le sens de circulation d'air dans le système 1. Les voies d'admission 6 sont par exemple placées sous la partie d'assise des passagers de l'arrière du véhicule V.

Une disposition, en bas dans l'habitacle intérieur HV, du préfiltre 11 peut permettre de concevoir un tiroir ou boîtier extractible sous des sièges pour retirer les particules les plus grossières arrêtées par le préfiltre, par exemple en nettoyant le préfiltre 11 qui est du type lavable. Eventuellement, le préfiltre 11 est disposé en bas, ici nettement sous le niveau des fenêtres W, dans un bloc sélectivement extractible en étant décalé sur un côté et/ou sous une partie d'assise prévue dans l'habitacle HV.

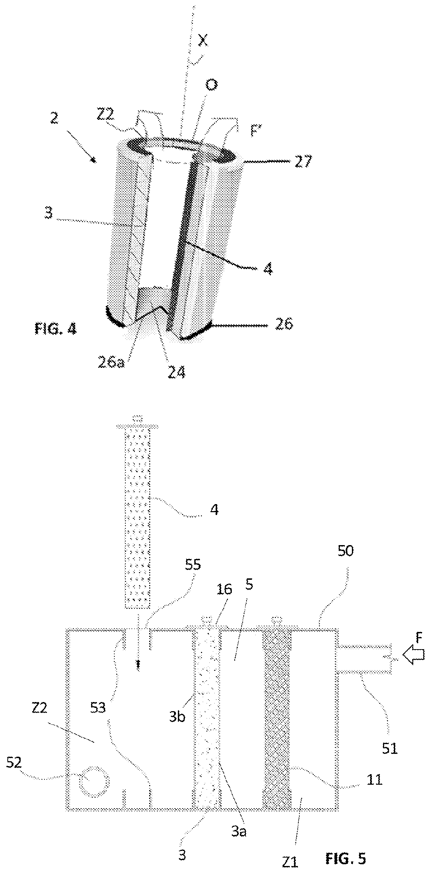

La figure 5 montre un exemple dans lequel un préfiltre 11 peut être retiré sélectivement, indépendamment de l'agencement filtrant 2 prévu pour éliminer les particules fines et satisfaisant le niveau H13. Dans ce cas non limitatif, le préfiltre 11 peut présenter un cadre de support et est monté parallèlement au média filtrant 3 d'un élément filtrant amovible dans un boîtier 50. Au moins une ouverture d'accès 55 peut permettre d'extraire l'agencement 2. L'élément ou agencement filtrant 2 est ici fixé coulissant et extractible suivant une direction d'extraction. La manipulation peut être facilité en rendant le média 3 solidaire d'une partie de couvercle ou portion préhensible 16 analogue. Le détachement de cet élément 2 peut être facilité en utilisant un cadre ou flasque de support pour le maintien du média filtrant 3 qui recouvre une extrémité axiale du média qui est une extrémité proximale/proche de l'ouverture de boîtier par laquelle l'élément peut être extrait. Le préfiltre 11 peut aussi être prévu dans un logement séparé, délimité par une canalisation ou un boîtier différent du boîtier 50 définissant la chambre de filtration 5.

La figure 4 montre une option dans laquelle l'agencement 2 est sous forme d'élément filtrant amovible pouvant se loger dans un boîtier de filtre réutilisable, et ayant une configuration annulaire du média 3. Au moins un support axial, sous forme de flasque maintient le média filtrant 3 purificateur d'air dans une configuration annulaire autour d'un axe central X, ce qui permet une filtration d'air centripète. La couche 4 additionnelle, ici prévue par exemple pour la captation de composés volatils s'étend intérieurement par rapport au média filtrant 3, en s'étendant autour d'un espace creux 24. Dans le boîtier, l'espace creux 24 forme typiquement une partie de la zone d'aval Z2 de la chambre de filtration 5. L'espace creux 24 n'est pas accessible par une extrémité (axiale) qui est obturée, ici, par une portion centrale de fermeture 26a du flasque 26 qui recouvre l'extrémité annulaire du média 3.

Eventuellement, il est prévu un boîtier de filtre ayant un bol qui présente une ouverture recouverte par un couvercle déplaçable lors d'une opération de rechange de l'agencement 2. Alternativement, le boîtier 50 peut être du type scellé, avec un couvercle plastique soudé par exemple. Ceci évite le risque d'échappement de particules nocives lors de manipulations pendant l'opération de rechange. Dans un tel cas, le boîtier de filtre peut disposer d'un raccord rapide à son entrée et à sa sortie, respectivement pour la connexion fluidique à une conduite d'amenée de la structure 1a et pour la connexion fluidique à une tubulure de la conduite 7 évacuant l'air purifié vers des zones de circulation supérieure, via les branches 7a, 7b.

Plus généralement, on comprend que le boîtier 50 peut être adapté pour recevoir un élément ou agencement filtrant 2 avec au moins un média filtrant 3 lui permettant de remplir le niveau de séparation de classe H13. Un montage de cet élément 2 peut être réalisé de façon amovible et en formant une zone de contact d'étanchéité (typiquement annulaire) entre un support rigide 26, 27 ou 53 associé au média 3 et une paroi interne du boîtier 50, afin de séparer la zone d'amont Z1 de la zone d'aval Z2. En pratique lors du fonctionnement normal du système 1, l'air admis dans la zone d'amont Z1 est orienté sur une face 3a du média 3 (face d'entrée) et ne peut parvenir dans la zone d'aval Z2 qu'en passant par la face de sortie 3b.

Le média filtrant 3 se présente par exemple sous la forme d'une couche de média filtrant, en étant plié, typiquement à intervalles régulier, pour former un média filtrant plissé. Un support tubulaire rigide peut être prévu contre la face intérieure du média 3 ou d'une couche plus en aval, ce support pouvant s'étendre entre deux flasques 26, 27 dont l'un a une ouverture centrale O formant un débouché axial de l'espace interne creux 24, délimité par la face la plus interne du média annulaire où circule de l'air purifié (voir sur la figure 4 par exemple). Dans des configurations plissées, il existe une pluralité de lignes de pliage parallèles entre elles, afin d'accroître la surface du média filtrant 3 exposée au flux d'air à filtrer. Des sous-couches de nature différente peuvent optionnellement composer la couche de média filtrant 3. Dans des modes de réalisation, le média filtrant 3 est du type soumis à un traitement thermique, de sorte qu'il conserve le plissage en raison d'un effet de durcissement.

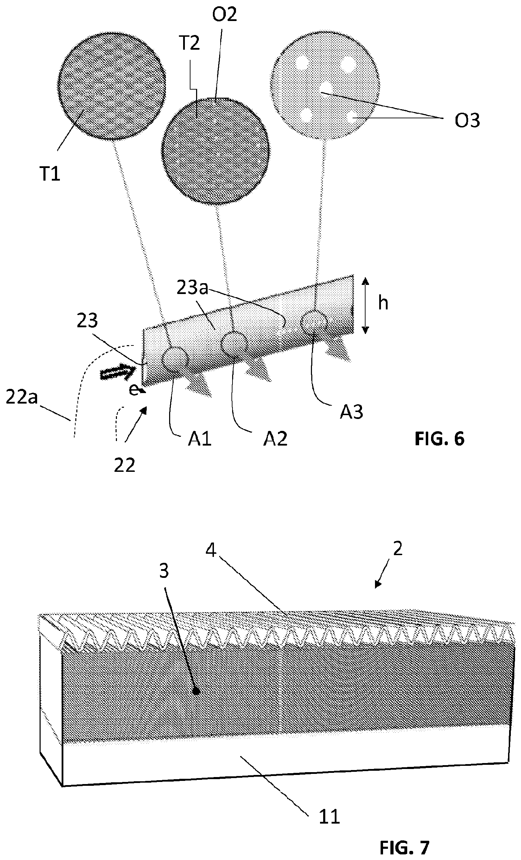

En référence à la figure 7, on peut voir que la couche 4 peut-être du type plissé. Ici il a été représenté trois couches différentes rassemblées en un seul bloc constitutif de l'agencement de filtration 2. Comme bien visible ici, un préfiltre 11 peut être inclus dans le bloc, de sorte que ce préfiltre 11 est agencé en amont du média 3 tout en étant logé dans la même chambre de filtration. Le média 3 peut constituer tout ou partie de l'élément filtrant 2, en ayant par exemple à lui seul un niveau H13. L'une au moins des autres couches peut être plissée. Dans le cas de la figure 7, on a représenté une première couche qui peut constituer le préfiltre 11. La couche formant le préfiltre 11 peut être détachable et/ou lavable. Lorsque le préfiltre 11 est conçu séparable des autres couches, que ce soit dans une configuration d'agencement filtrant en panneau (comme ça peut être le cas avec la conception montrée sur la figure 7) ou non, il peut alors être réutilisé sélectivement après un traitement et/ou un lavage.

Eventuellement, une conception avec un conduit et/ou orifice de dérivation associé à un clapet unidirectionnel, peut être prévue pour l'un parmi le préfiltre 11 ou l'agencement filtrant 2, s'il existe un risque de colmatage. Par exemple, si de l'eau passe avec l'air F' et qu'un état de surpression relative est obtenu en amont du préfiltre 11, le ou les clapets peuvent libérer des accès secondaires à la zone Z1 d'amont de ma chambre de filtration 5. Le cas échéant, une zone d'impaction et/ou une grille additionnelle de filtration primaire du flux peut être prévue dans la canalisation secondaire permettant une telle dérivation.

Le système 1, constituant un purificateur d'air pour l'habitacle HV, peut présenter une partie de collection et d'aspiration d'air, par exemple en formant une bouche ou structure 1a avec conduit 6 d'aspiration rectangulaire, ayant une longueur correspondant à une largeur de l'habitacle HV, comme dans le cas de la figure 3 par exemple. Alternativement, des conduits d'aspiration sont distribués régulièrement avec des écartements dans le sens de la largeur et/ou dans la direction d'arrière en avant Ar-Av.

Sur la figure 3, on a représenté un moyen 8 de mise en circulation d'air, par exemple une pompe à air séparée ou un ventilateur, séparé du ou des conduits d'entrée et placé dans un tronçon de circulation commun à plusieurs zones/voies d'entrée. Le moyen 8 d'aspiration peut être fixé à une partie ou face supérieure de la structure inférieure 1a du système 1 munie du conduit 6. Le tuyau de guidage 12 associé aux conduit(s) ou voie(s) 6 relie ici entre eux la structure 1a inférieure et la partie de traitement 10. Des parties ou pattes de fixation/assemblage MF sont solidaires de la structure inférieure 1a et peuvent être formées par pliage d'une plaque ou tôle métallique, rapportés de façon similaire, ou intégrés/moulés d'une pièce avec une pièce de canalisation d'air appartenant à la structure 1a.

Plus généralement, on comprend que la structure inférieure 1a du système peut être fixée à l'arrière du véhicule et au-dessous d'une partie d'assise, à l'aide des parties de fixation MF, et ainsi la fente 1f ou autre zone d'admission peut recevoir/prélever un flux d'air F à purifier dans une zone basse de l'habitacle, ce flux pouvant circuler dans la direction d'avant en arrière Av-Ar.

En complément ou indépendamment de ce qui précède, le moyen 8 dispose d'un moteur et est relié à un appareil électrique du véhicule, de façon à être alimenté électriquement. L'appareil peut être associé à une batterie/système d'accumulation d'énergie similaire du véhicule. Dans des variantes, le moyen 8 de mise en circulation d'air est apte à être alimenté électriquement de façon autonome, éventuellement par une batterie associée à un capteur solaire monté sur le véhicule, en particulier dans une partie arrière du toit 9 du véhicule.

Dans des options de réalisation, le système 1 est rendu actif en démarrant le moyen 8 dès que le véhicule V démarre ou même avant dans une option avec programmation d'une purification par anticipation. Lorsque le véhicule V démarre, une énergie peut être fournie au ventilateur ou moyen 8 similaire de sorte que le système 1 purificateur d'air puisse immédiatement aspirer le l'air F stagnant en partie basse de l'habitacle HV. Dans certaines réalisations, la structure inférieure 1 peut aussi présenter des modules d'aspiration avancés, situés plus en avant dans l'habitacle HV, et raccordés à la partie de traitement 10.

Le moyen 8 de mise en circulation d'air peut être disposé entre l'agencement filtrant 2 et la conduite d'évacuation 7 qui achemine l'air purifié dans les moyens de diffusion 22 pour la diffusion d'air purifié dans une zone supérieure de l'habitacle HV. Ainsi, le moyen 8 peut être directement raccordé à la sortie 52. Alternativement, le moyen 8 peut être prévu en amont de l'agencement filtrant 2, par exemple en formant une pompe à air. Dans des options préférées, le moyen 8 est adjacent à une extrémité (de préférence l'extrémité inférieure) de la conduite 7 qui s'étend vers le haut et/ou est prolongée vers le haut par des branches 7a, 7b canalisant chacune l'air purifié F' obtenu au niveau de la sortie 52 du boîtier 50 ou zone d'évacuation similaire en communication avec la zone d'aval Z2.

Plus généralement, il est prévu une voie d'évacuation d'air ascendante qui rejoint le plafond et permettant de distribuer l'air purifié dans le haut de l'habitacle le long du toit 9 du véhicule V, par le plafond et/ou en position adjacente au plafond. Les branches 7a, 7b peuvent se rejoindre dans une zone montante suivant la direction de bas en haut B-H ou se rejoindre en haut de l'habitacle, le long du plafond. En variante, les branches 7a, 7B peuvent se connecter à la partie de traitement 10 sensiblement au même niveau de hauteur que l'évacuation 7 ou la sortie 52.

En référence à la figure 2, il peut être prévu deux branches 7a, 7b principales de diffusion réparties sur les côtés (latéralement dans le véhicule V et/ou dans des zones de marge latérales du plafond) et allant de l'arrière vers l'avant.

### Exemples de moyens de diffusion

Les moyens 22 de diffusion peuvent être allongées, suivant la direction arrière-avant Ar-Av et présenter des perforations et/ou zones perméables à l'air 23a qui sont aussi bien prévues en position proximale P1 d'une extrémité arrière 9a du toit que dans une position distale P2 pouvant correspondre à l'extrémité avant 9b du toit 9. La diffusion réalisée peut être douce, en évitant de concentrer le flux d'air dans des ouvertures d'accélération du flux d'air. Une ou plusieurs gaines 24a, 24b perméables au gaz peuvent faire partie de ces moyens 22 de diffusion. De telles gaines 24a, 24b peuvent inclure chacune un passage 23, par exemple d'extension longitudinale, pour acheminer l'air purifié issu de la chambre de filtration 5 jusque dans des zones textiles des gaines, permettant la diffusion douce dans l'habitacle HV avec un apport diffus d'air qui peut alors descendre ensuite vers le ou les passagers.

Comme visible sur la figure 2, le système purificateur 1 peut présenter deux parties espacées/distribuées, pour occuper une zone latérale droite 20a de diffusion et une zone latérale gauche 20b de diffusion. On comprend aussi, comme illustré par exemple en figure 1, que ces deux zones 20a, 20b sont verticalement éloignées/significativement plus haute que des zones d'arrivée d'air extérieur de l'équipement 20 de type HVAC.

Dans des réalisations, les moyens 22 de diffusion d'air purifié comprennent au moins deux zones ou régions de diffusion agencées en série ou en parallèle dans un circuit où est acheminé l'air purifié provenant de la zone d'aval Z2 (dans la chambre 5). Ces régions peuvent être séparées l'une de l'autre par un dispositif d'aiguillage 36 présentant au moins une vanne réglable, afin d'ajuster par exemple un débit de diffusion d'air sélectivement en fonction desdites zones/régions de diffusion. Dans ce cas, il est permis optionnellement de définir deux régions de diffusion :
- l'une correspondant à la zone avant de diffusion du système 1, associée à un premier rang d'assise du véhicule V, et
- l'autre correspondant à une autre des deux zones de diffusion et agencée pour former une zone arrière de diffusion, associée à un deuxième rang d'assise du véhicule.

Sur la figure 2, on peut voir par exemple une conduite 38 de dérivation permettant au flux d'air purifié acheminé via la conduite 7 de sauter un tronçon (arrière ici) des moyens 22 de diffusion, en rejoignant la zone avant de diffusion. Selon le réglage, on comprend que le ou les tronçons en partie arrière de l'habitacle HV peuvent être alimentés avec une fraction plus ou moins élevée du flux d'air ascendant acheminé jusqu'au dispositif d'aiguillage. Une position du clapet pour obturer la conduite de dérivation peut optionnellement permettre ou être compatible avec un écoulement d'air en série dans les tronçons respectifs assurant la diffusion d'air purifié dans l'habitacle intérieur HV, éventuellement avec un ou des passage 23 guidant l'air d'arrière en avant suivant la direction générale Ar-Av.

Le système 1 de purification de l'air d'habitacle peut optionnellement se commander à distance, par une télécommande dédiée ou par un terminal de communication 15 tel qu'un téléphone disposant d'une application ou logiciel adapté. Un utilisateur peut paramétrer via l'application, une commande pour transmettre un signal/ordre d'actionnement du moyen de mise en circulation d'air, le cas échéant préprogrammé avec un horodatage. Dans ce cas, le système présente un module M disposant d'une fonction ou ligne de réception, pour la communication avec le terminal 15 qui peut être un terminal distant. Une liaison sans fil peut être prévue pour cela.

Au moins un paramètre représentatif d'un débit de circulation dans les moyens de diffusion peut être ajusté grâce à une interface de contrôle, par exemple une interface graphique utilisateur, dont dispose le terminal 15. De cette façon, il est permis pour l'utilisateur de commander une phase de purification, éventuellement avant un début de conduite envisagée pour le véhicule V équipé du système 1. Ce mode d'activation est compatible avec un fonctionnement autonome du système 1, typiquement sans déclencher l'équipement HVAC prévu pour insuffler de l'air extérieur dans l'habitacle. Une batterie adéquate, par exemple rechargeable et/ou liée à une batterie disponible dans le véhicule, peut être utilisée lors de ce mode d'épuration, que ce mode soit déclenché ou non à l'état avant démarrage du véhicule V.

Dans des options, les moyens 22 de diffusion peuvent présenter des gaines permettant une circulation d'air dans au moins deux ou trois directions dans des tronçons respectifs qui peuvent être rectilignes ou ondulés. Ces gaines 24a, 24b, éventuellement souples, sont conçues ou conformées pour former des boucles ou un agencement en serpentin. Ceci peut permettre un allongement du parcours de l'air purifié circulant dans une gaine avec un débit décroissant au fur et à mesure de la diffusion à travers la paroi de gaine perméable/rendue perméable par des microperforations.

Indépendamment de la façon dont peut être contrôlé ou non le débit d'air purifié F', il est par exemple prévu que le système 1 soit pourvu seulement d'un circuit de circulation d'air, en provenance de la chambre de filtration 5, qui fonctionne de façon autonome par rapport à l'équipement 20. La chambre de filtration 5, la conduite d'évacuation 7 avec les éventuelles branches 7a, 7b pour atteindre au moins une région supérieure de circulation d'air, et les passages 23 périphériques de circulation d'air formés depuis ces branches 7a, 7b font ainsi partie d'un même circuit. Les moyens 22 de diffusion peuvent consister en des portions plates recouvrant des portions de paroi de l'habitacle HV et qui forment des canalisations dont la section en profil est dissymétrique, en particulier pour être perméable seulement du côté orienté vers l'habitacle intérieur HV, donc vers le bas ou vers une zone plus centrale de cet habitacle.

Le recours à des gaines 24a, 24b, en particulier des gaines au moins en partie textiles, permet de préassembler les moyens diffusion 22 ou tout du moins de réaliser un seul bloc allongé de diffusion. La gaine 24a, 24b peut être éventuellement coudée ou pourvues de retours, comme illustré sur l'exemple non limitatif de la figure 2. Optionnellement, chaque gaine 24a, 24b peut s'étendre vers l'avant, au moins dans un tronçon dit supérieur ou principal, depuis une extrémité arrière, sensiblement à un même niveau de hauteur au-dessus de zones de vitrage/fenêtres. La gaine 24a, 24b est ainsi sensiblement horizontale dans un tel tronçon principal de diffusion.

En outre, chaque gaine 24a, 24b, peut disposer d'une portion d'entrée de gaine 22a, de préférence disposée latéralement dans l'habitacle HV, contre un élément de paroi extérieure du véhicule V. La portion 22a forme un tronçon de circulation ascendante d'air. Une fraction d'air purifié est par exemple diffusée à travers l'épaisseur de cette portion d'entrée 22a, optionnellement en traversant le matériau textile et/ou via des microperforations O2 ou perforations O3 de cette portion d'entrée 22a. Le complément de cette fraction circule dans le tronçon principal, où l'air purifié est également diffusé.

En référence à présent à la figure 6, les gaines 24a, 24b présentent des perforations et/ou des zones perméables à l'air 23a qui forment ou sont intégrées dans l'épaisseur e du matériau T1, T2 des gaines 24a, 24b, par exemple sans créer localement de surépaisseur et/ou sans créer un effet d'accélération dans une ouverture de section réduite bordée de façon annulaire par une section de paroi imperméable à l'air. Deux tronçons de gaine textile, continus, sont par exemple fixés de manière permanente ou de façon amovible contre une face interne de la paroi externe d'habitacle s'étendant le long du bord supérieur des fenêtres W du véhicule V. Les tronçons sont allongés, avec une longueur de gaine qui peut être supérieure à 0,5 mètre par exemple, tandis que l'extension h en largeur de la gaine peut être limitée à moins de 0,2 mètres, par exemple de l'ordre de 5 mm ou 10 mm seulement lorsque plusieurs gaines sont prévues dans les moyens 22 de diffusion.

Les tronçons des gaines ont chacun une extrémité, commune ou non, formant une entrée. Ils peuvent se raccorder, par exemple annulairement et avec chevauchement, à un raccord rigide formant une extrémité terminale haute d'une partie ascendante des conduites ou branches 7, 7a, 7b, de sorte que l'air purifié depuis la partie de traitement 10 positionnée plus bas parvienne dans les passages 23 longitudinaux de ces gaines 24a, 24b et soit diffusé radialement du fait de la structure perméable de ce gaines 24a, 24b.

Comme bien visible sur la figure 6, le textile T1 constitutif de tout ou partie des gaines peut être conçu avec des microperforations qui permettent une perméabilité uniforme pour laisser passer l'air. Du polyester peut faire partie :
- du textile T1 qui présente une structure limitant le débit d'air à un premier seuil, par exemple sans perforations visibles,
- ou du textile T2 qui présente en outre des passages ou microperforations, ici sous forme de trous fond O2, ayant une même orientation de diffusion (obtenue par microperforation directionnelle, avec des trous fins O2 de taille submillimétrique) et limitant le débit, par exemple en dessous d'un autre seuil qui est supérieur à celui prévu pour le textile T1.
Le textile T1, T2, peut être imperméable à l'eau tout en étant respirant. En complément ou en alternative, il peut inclure différentes fibres ou couches de matériau.

Le textile T1 peut être considéré respirant en laissant l'air purifié F' traverser chaque portion de textile, dont l'épaisseur e reste par exemple inférieure à 3 ou 4 mm (épaisseur mesurée entre les faces du textile T1 : celle qui délimite et/ou est orienté vers le passage 23, et celle tournée à l'opposé vers l'habitacle HV). Plus généralement les gaines peuvent présenter une certaine souplesse localement. Un revêtement perméable à l'air (éventuellement poreux) de rigidification, par exemple en matériau rigide et hydrophobe, peut éventuellement recouvrir la gaine textile sans limiter/réduire le débit transmis via les gaines 24a, 24b. Ce revêtement peut être prévu uniquement du côté habitacle/côté accessible des moyens 22 de diffusion (côté habitacle intérieur HV).

Les moyen 22 de diffusion d'air peuvent présenter une variété dans la structure des zones perméables à l'air 23a, par exemple en ayant des régions A1 minimisant l'effet directionnel du débit, sans perforation visible, mais aussi des orifices ou trous fins O2 dans au moins une région A2 complémentaire pouvant faire partie d'une gaine textile. D'autres régions A3 peuvent éventuellement être compatibles avec la présence d'orifice O3 de taille millimétrique, plus grands que les trous fins O2.

En référence aux figures 1 et 2, le système 1 est utilisable en association avec l'équipement 20 qui assuré la dépollution de l'air provenant de l'extérieur à l'aide du filtre 25. Au moins deux couches de filtration différentes sont prévues dans ce filtre 25 : une première couche pour filtrer les particules solides et une deuxième couche pour permettre de filtrer des gaz nocifs, par exemple une couche incluant du charbon actif, permettant de neutraliser les gaz nocifs (et typiquement les gaz malodorants).

Dans des options, une couche de microfibres (par exemple des microfibres ultrafines) du filtre 25 est apte à séparer à plus de 98 % les poussières fines et les particules de suie pouvant se trouver en suspension dans l'air extérieur. Le filtre 25 peut disposer également d'une couche antimicrobienne spécifique agissant contre les virus et les bactéries, par exemple de niveau H13 ou mieux, et qui empêche optionnellement le développement des moisissures et fixe les allergènes et le pollen dans la couche filtrante.

Une installation pour véhicule automobile peut inclure l'équipement 20 pourvu du filtre 20 et le système 1 de purification de l'air intérieur, autonome/indépendant de l'équipement 20. Le système dispose au moins de trois étages de filtration (dont le préfiltre 11 et le média 3 purificateur de niveau H13), pouvant tous être disposés sous des sièges arrière du véhicule V dans des options de réalisation. Le média 3, protégé par le préfiltre 11, s'avère efficace pour capter les particules ultrafines ainsi que les bio contaminants aéroportés comme les bactéries, les champignons, les virus et les pollens. Dans certaines options, il est prévu une couche 4 incluant du charbon actif à la fois dans l'équipement 20 et dans le système 1, cette couche pouvant éventuellement être identique ou pourvue du même composant adsorbant dans l'une et l'autre des parties de traitement d'air 10, 25.

Dans une forme de réalisation, le toit du véhicule est recouvert par un ensemble textile, par exemple rapporté d'un seul tenant, qui inclut les gaines textiles microperforées. Cet ensemble peut être prolongé sur les côtés par deux portions d'entrée 22a, opposées et formées latéralement. Ces portions d'entrée 22a forment des raccords pour raccorder les moyens 22 de diffusion à la conduite 7 qui peut s'étendre en hauteur avec une extension verticale inférieure au différentiel de hauteur entre la sortie 52 et le niveau de l'ensemble textile plat formé le long du toit.

Sur la figure 1, on a illustré une variante avec une extrémité haute 7c d'une branche pouvant former la partie de canalisation ascendante de l'air purifié, sur laquelle se raccordent les moyens 22 de diffusion. Dans ce cas, la diffusion d'air purifié n'est réalisée que dans la ou les parties supérieures du système 1, avec une diffusion d'air depuis le haut de l'habitacle HV, de préférence latéralement et au-dessus des portes du véhicule V.

Entre la zone basse de ventilation (verticalement distale du toit 9) incluant le moyen 8 de mise en circulation d'air et une telle partie supérieure pour la diffusion descendante d'air purifié, on peut au choix diffuser ou non, par des portions latérales formant les portions d'entrée 22a, une fraction de l'air purifié qui sort de la chambre 5 dans une sortie 52 de boîtier 50 qui peut être située dans ou adjacente à la zone basse de ventilation.

Dans le système montré sur la figure 1, on a représenté des organes 17 de diffusion pour la diffusion d'au moins un produit sec, volatile, par exemple une composition à effet biocide. Ces organes 17 sont situés au même niveau que la ou les parties supérieures du système 1 qui participent à la diffusion d'air purifié. Plus généralement, on peut prévoir que les moyens 22 de diffusion associent une diffusion d'air purifié (ici après l'utilisation de gaines perméables 24a, 24b) et une distribution, par exemple sous forme d'une dose calibrée délivrée par une buse ou un injecteur constitutif d'un organe 17. Lorsqu'il est prévu un ensemble d'un bloc pour former la ou les parties supérieures de diffusion d'air purifié, on peut prévoit de distribuer les organes 17 au travers de ce bloc. En variante, les organes 17 de diffusion pour le produit volatile peuvent être montés adjacents ou espacés des gaines 24a, 24b.

Un ou plusieurs produits secs volatiles, choisi(s) parmi un composant biocide et une fragrance parfumée, peuvent être issus d'un réservoir embarqué. Chacun des organes 17 de diffusion peut être alimenté depuis un tel réservoir en étant connecté (par une connexion fluidique étanche) avec une conduite associée au réservoir correspondant de produit. Eventuellement, il est prévu au moins un organe 17 de diffusion, supporté par ou rendu solidaire d'une gaine appartenant aux moyens 22 de diffusion d'air purifié.

La partie de traitement 10 peut consister en un dispositif, éventuellement portatif et/ou dont les éléments sont colocalisés dans un même contenant, avec la possibilité de changer un composant/élément filtrant de façon sélective, sans démontage du côté de l'équipement 20 de climatisation (équipement HVAC). Ceci peut permettre un gain de temps pour immobiliser le véhicule le moins longtemps possible.

Le système de purification peut s'adapter de façon simple à une large variété d'habitacles intérieurs, sans modification de l'équipement 20 de climatisation. L'assainissement de l'air ainsi réalisé peut s'accompagner d'une diffusion d'air particulièrement douce en délivrant l'air purifié de manière efficace puisque les moyens 22 de diffusion sont placés le long du/dans le plafond du véhicule. Le dimensionnement des moyens 22 de diffusion est simple à adapter en fonction du volume de l'habitacle HV. Le système 1 de purification peu typiquement permettre de recycler l'air 5 fois par heure. La partie de traitement 10 cumule des couches complémentaires qui se combinent pour permettre de réintroduire et diffuser de l'air purifié et sans odeur incommodante.

En outre, ce système 1 de ventilation et purification n'encombre pas l'espace disponible à l'intérieur du véhicule puisque seul les conduits ou branches analogues incluant les gaines peuvent être installées dans ou contre le plafond. L'option avec des gaines 24a, 24b, en particulier à structure textile, offre une excellente diffusion d'air purifié, compatible avec une homogénéité des températures, avec un apport d'air purifié compatible avec une dé-stratification dans la mesure ou l'air recirculé via le système 1 provient d'une zone basse qui n'est généralement pas aussi chaude que dans la zone supérieure de l'habitacle intérieur HV, avant la mise en fonctionnement du système 1.

Il doit être évident pour les personnes versées dans l'art que la présente invention permet des modes de réalisation sous de nombreuses autres formes spécifiques sans l'éloigner du domaine d'application de l'invention comme revendiqué.

## Revendications

1. Système (1) de purification de l'air d'un habitacle (HV) pour passager et/ou conducteur, en particulier d'un habitacle automobile, le système comprenant :
- un agencement filtrant (2) incluant un média filtrant (3) purificateur d'air séparant au moins les classes de particules fines PM0,3 et PM0,1, ledit média filtrant (3) étant de préférence de classe H13, et au moins une couche (4) pour la captation de composés volatils, en particulier par adsorption moléculaire ;
- une chambre de filtration (5) dans laquelle s'étend le média filtrant (3) purificateur d'air de sorte que l'agencement filtrant (2) délimite une zone d'amont (Z1) en communication avec une ou plusieurs voies d'admission (6) du système (1) ;
- un moyen (8) de mise en circulation d'air pour faire circuler l'air (F) issu de l'habitacle (HV) dans une partie de traitement d'air (10) incluant l'agencement filtrant (2) et une conduite (7) d'évacuation d'air purifié par l'agencement filtrant (2), sachant que le moyen (8) de mise en circulation d'air permet de refouler, dans la conduite d'évacuation (7), l'air purifié (F') circulant dans une zone d'aval (Z2) de la chambre de filtration (5) ;
- un préfiltre (11) pour capter des particules solides et/ou liquides en amont du média filtrant (3) suivant un sens de circulation de l'air sélectivement permis par le moyen (8) de mise en circulation d'air ;
dans lequel la conduite d'évacuation (7) est en communication avec au moins une branche, de préférence deux branches (7a, 7b), la conduite et/ou chaque branche formant une canalisation ascendante en communication avec des moyens (22) de diffusion d'air purifié qui s'étendent, depuis une extrémité haute (7c) de la branche, vers l'avant de l'habitacle automobile (HV),
et dans lequel les moyens (22) de diffusion d'air purifié comprennent au moins une gaine (24a, 24b), longeant le toit (9) de l'habitacle (HV) de façon à diffuser l'air purifié de façon descendante dans l'habitacle, chaque gaine (24a, 24b) présentant des perforations et/ou zones perméables à l'air (23a) qui sont aussi bien prévues en position proximale (P1) d'une extrémité arrière (9a) du toit que dans une position distale (P2), au niveau de laquelle ladite gaine (24a, 24b) recouvre par l'intérieur tout ou partie d'une extrémité avant (9b) du toit.

2. Système selon la revendication 1, dans lequel les moyens (22) de diffusion d'air purifié délimitent des passages (23) de circulation d'air périphériques par rapport à une zone d'occupation de passagers dans l'habitacle,
et dans lequel la chambre de filtration (5), la conduite d'évacuation (7) et les passages périphériques de circulation d'air font partie d'un même circuit de circulation d'air indépendant et séparé d'un équipement (20) de climatisation et contrôle de température par filtration et conditionnement d'un air prélevé à l'extérieur de l'habitacle, le moyen (8) de mise en circulation d'air étant configuré pour être placé dans une région arrière de l'habitacle par référence à un sens de circulation dit vers l'avant du véhicule (V) définissant l'habitacle (HV).

3. Système selon la revendication 1 ou 2, dans lequel les perforations et/ou zones perméables à l'air (23a), pour chaque gaine (24a, 24b), sont intégrées dans une épaisseur (e) de matériau de gaine, de préférence sans former de reliefs saillants sur la surface externe de la gaine.

4. Système selon l'une quelconque des revendications précédentes, dans lequel la partie de traitement d'air (10) comprend un boîtier (50) qui délimite la chambre de filtration (5) et est pourvu d'une entrée (51), formant tout ou partie de la ou des voies d'admission (6), pour faire circuler de l'air admis dans la zone d'amont (Z1) de la chambre de filtration, la zone d'aval (Z2) étant :
- délimitée par un élément filtrant préassemblé incluant le média filtrant (3) purificateur d'air ; et
- en communication avec la conduite d'évacuation (7) par l'intermédiaire d'une zone basse de ventilation, verticalement distale du toit, équipée d'au moins un élément ventilateur qui constitue tout ou partie du moyen (8) de mise en circulation d'air.

5. Système selon l'une quelconque des revendications précédentes, dans lequel il est prévu deux tronçons de gaine textile (24a, 24b) qui sont continus, en formant tout ou partie de l'au moins une gaine, et qui s'étendent chacun continûment de l'extrémité arrière (9a) du toit jusqu'à l'extrémité avant (9b) du toit en définissant chacun une section utile de diffusion d'air purifié, de préférence supérieure à un seuil, par exemple avec une section utile de diffusion au moins égale à 4 dm².

6. Système selon l'une quelconque des revendications précédentes, dans lequel le média filtrant (3) purificateur d'air s'étend de façon annulaire autour d'un axe central (X) et est conçu pour permettre une filtration d'air centripète, la couche (4) pour la captation de composés volatils étant agencée intérieurement par rapport au média filtrant (3), en s'étendant autour d'un espace creux (24), l'espace creux (24) formant de préférence une partie de la zone d'aval (Z2) de la chambre de filtration (5).

7. Système selon l'une quelconque des revendications précédentes, dans lequel les moyens (22) de diffusion d'air purifié comprennent sélectivement des gaines souples, les gaines incluant une matière textile (T1, T2) définissant un revêtement extérieur de la gaine (24a, 24b), visible depuis l'intérieur de l'habitacle (HV) ou recouvert par une couche protectrice plus rigide que la gaine.

8. Système selon l'une quelconque des revendications précédentes, dans lequel le préfiltre (11) est configuré pour séparer des particules de diamètre ou taille caractéristique submillimétrique, le préfiltre (11) étant disposé au niveau d'une entrée d'admission du système qui :
- présente au moins une fente allongée (1f) ou des ouvertures distribuées sur une largeur de l'habitacle (HV) ;et
- est situé sous une partie d'assise des sièges arrière et/ou entièrement sous le niveau d'ouvertures de fenêtre (W) obturées par des vitres.

9. Système selon l'une quelconque des revendications précédentes, dans lequel les moyens (22) de diffusion d'air purifié disposent d'un ou plusieurs organes (17) de diffusion pour la diffusion d'au moins un produit volatile, sec, choisi parmi un composant biocide et une fragrance parfumée, chacun des organes (17) de diffusion étant en connexion fluidique avec un réservoir correspondant de produit, les organes (17) de diffusion étant de préférence supportés par ou rendus solidaires des gaines (24a, 24b).

10. Utilisation combinée d'un agencement filtrant (2) et de moyens (22) de diffusion d'air purifié dans un habitacle (HV) pour passager et/ou conducteur, dans un véhicule (V), **caractérisée en ce que** l'agencement filtrant (2) et les moyens (22) de diffusion d'air purifié sont interconnectés à l'arrière du véhicule pour former le système (1) de purification de l'air selon l'une quelconque des revendications précédentes, ce grâce à quoi l'air purifié diffusé au travers de gaines (24a, 24b) des moyens (22) de diffusion d'air, dans différentes positions adjacentes au toit (9) du véhicule (V) suivant la direction d'avant en arrière du véhicule, est exempt de particules fines PM0,3 et PM0,1.

11. Utilisation selon la revendication 10, dans laquelle la totalité des composants de filtration et des composants actifs pour l'épuration et la mise en circulation de l'air du système (1) sont situés dans une partie arrière (42) du véhicule (V) en permettant un fonctionnement du système indépendant du fonctionnement d'un équipement (20) de climatisation intégré à l'avant du véhicule (V) qui est configuré pour réaliser la climatisation et le contrôle de température par filtration et conditionnement d'un air prélevé à l'extérieur de l'habitacle (HV).

12. Utilisation selon la revendication 10 ou 11, dans laquelle les moyens (22) de diffusion d'air purifié comprennent au moins deux zones de diffusion séparées l'une de l'autre par un dispositif d'aiguillage (36) présentant au moins une vanne réglable, afin d'ajuster un débit de diffusion d'air sélectivement en fonction desdites zones de diffusion, avec de préférence une des deux zones de diffusion agencée pour former une zone avant de diffusion, associée à un premier rang d'assise du véhicule et une autre des deux zones de diffusion agencée pour former une zone arrière de diffusion, associée à un deuxième rang d'assise du véhicule.

## Patentansprüche

1. Luftreinigungssystem (1) für einen Innenraum (HV) für Passagiere und/oder einen Fahrer, insbesondere einen Kraftfahrzeugraum, wobei das System Folgendes umfasst:
- eine Filteranordnung (2), die ein luftreinigendes Filtermedium (3), das mindestens die Feinstaubklassen PM0,3 und PM0,1 abscheidet, wobei das Filtermedium (3) vorzugsweise zur Klasse H13 gehört, und mindestens eine Schicht (4) zum Auffangen flüchtiger Verbindungen, insbesondere durch molekulare Adsorption, enthält;
- eine Filterkammer (5), in der sich das luftreinigende Filtermedium (3) so erstreckt, dass die Filteranordnung (2) einen vorgelagerten Bereich (Z1) in Verbindung mit einem oder mehreren Einlasswegen (6) des Systems (1) abgrenzt;
- ein Luftzirkulationsmittel (8) zum Zirkulieren der aus dem Innenraum (HV) stammenden Luft (F) in einem Luftbehandlungsabschnitt (10), der die Filteranordnung (2) und eine Leitung (7) zum Abführen der durch die Filteranordnung (2) gereinigten Luft enthält, wobei das Luftzirkulationsmittel (8) es ermöglicht, die gereinigte Luft (F'), die in einem nachgelagerten Bereich (Z2) der Filterkammer (5) zirkuliert, in die Abluftleitung (7) zurückzufördern;
- einen Vorfilter (11) zum Auffangen von festen und/oder flüssigen Partikeln vor dem Filtermedium (3) in einer Luftströmungsrichtung, die durch das Luftzirkulationsmittel (8) selektiv ermöglicht wird;
wobei die Abluftleitung (7) mit mindestens einer Abzweigung, vorzugsweise zwei Abzweigungen (7a, 7b), in Verbindung steht, wobei die Leitung und/oder jede Abzweigung eine Steigleitung bildet, die mit Mitteln (22) zur Diffusion von gereinigter Luft in Verbindung steht, die sich von einem oberen Ende (7c) der Abzweigung nach vorne in den Kraftfahrzeuginnenraum (HV) erstrecken,
und wobei die Mittel (22) zur Diffusion von gereinigter Luft mindestens eine Verkleidung (24a, 24b) umfassen, die entlang des Daches (9) des Innenraums (HV) verläuft, um die gereinigte Luft nach unten in den Innenraum zu diffundieren, wobei jede Verkleidung (24a, 24b) Perforationen und/oder luftdurchlässige Bereiche (23a) aufweist, die sowohl in proximaler Position (P1) eines hinteren Endes (9a) des Daches als auch in einer distalen Position (P2), in der die Verkleidung (24a, 24b) ein vorderes Ende (9b) des Daches von innen ganz oder teilweise bedeckt, vorgesehen sind.

2. System nach Anspruch 1, wobei die Mittel (22) zur Diffusion von gereinigter Luft periphere Luftzirkulationsdurchgänge (23) in Bezug auf einen Fahrgastbereich im Innenraum begrenzen,
und wobei die Filterkammer (5), die Abluftleitung (7) und die peripheren Luftzirkulationsdurchgänge Teil desselben unabhängigen Luftzirkulationskreises sind, der von einer Klimaanlage (20) zur Temperaturregelung durch Filterung und Konditionierung einer von außerhalb des Innenraums entnommenen Luft getrennt sind, wobei das Luftzirkulationsmittel (8) so eingerichtet ist, dass es in einem hinteren Bereich des Innenraums in Bezug auf eine Strömungsrichtung, die in Richtung des vorderen Bereichs des Fahrzeugs (V) verläuft, angeordnet ist, die den Innenraum (HV) definiert.

3. System nach Anspruch 1 oder 2, wobei die Perforationen und/oder luftdurchlässigen Bereiche (23a) für jede Verkleidung (24a, 24b) in eine Dicke (e) des Verkleidungsmaterials integriert sind, vorzugsweise ohne hervorstehende Erhebungen auf der Außenfläche der Verkleidung zu bilden.

4. System nach einem der vorhergehenden Ansprüche, wobei der Luftbehandlungsabschnitt (10) ein Gehäuse (50) umfasst, das die Filterkammer (5) begrenzt und mit einem Eingang (51) versehen ist, der den oder die Einlasswege (6) ganz oder teilweise bildet, um die angesaugte Luft in den vorgelagerten Bereich (Z1) der Filterkammer zu leiten, wobei der nachgelagerte Bereich (Z2):
- durch ein vormontiertes Filterelement begrenzt ist, das das luftreinigende Filtermedium (3) enthält; und
- in Verbindung mit der Abluftleitung (7) über einen unteren Lüftungsbereich steht, der vertikal distal von dem Dach liegt und mit mindestens einem Lüfterelement ausgestattet ist, das das gesamte oder einen Teil des Luftzirkulationsmittels (8) bildet.

5. System nach einem der vorhergehenden Ansprüche, wobei zwei durchgehende Textilverkleidungsabschnitte (24a, 24b) vorgesehen sind, die die mindestens eine Verkleidung ganz oder teilweise bilden und sich jeweils durchgehend von dem hinteren Ende (9a) des Daches bis zu dem vorderen Ende (9b) des Daches erstrecken, wobei jeder von ihnen einen nutzbaren Querschnitt für die Diffusion von gereinigter Luft definiert, der vorzugsweise größer als ein Schwellenwert ist, beispielsweise mit einem nutzbaren Diffusionsquerschnitt von mindestens 4 dm².

6. System nach einem der vorhergehenden Ansprüche, wobei sich das luftreinigende Filtermedium (3) ringförmig um eine Mittelachse (X) herum erstreckt und so ausgebildet ist, dass es eine zentripetale Luftfiltration ermöglicht, wobei die Schicht (4) zum Auffangen flüchtiger Verbindungen innerhalb des Filtermediums (3) angeordnet ist und sich um einen Hohlraum (24) herum erstreckt, wobei der Hohlraum (24) vorzugsweise einen Teil des nachgelagerten Bereichs (Z2) der Filterkammer (5) bildet.

7. System nach einem der vorhergehenden Ansprüche, wobei die Mittel (22) zur Diffusion von gereinigter Luft selektiv flexible Verkleidungen umfassen, wobei die Verkleidungen ein Textilmaterial (T1, T2) enthalten, das eine äußere Beschichtung der Verkleidung (24a, 24b) definiert, die von der Innenseite des Innenraums (HV) sichtbar ist oder von einer Schutzschicht bedeckt ist, die steifer ist als die Verkleidung.

8. System nach einem der vorhergehenden Ansprüche, wobei der Vorfilter (11) so eingerichtet ist, dass er Partikel mit einem submillimetrischen charakteristischen Durchmesser oder einer submillimetrischen charakteristischen Größe abscheidet, wobei der Vorfilter (11) an einem Eingang des Systems angeordnet ist, der:
- mindestens einen länglichen Schlitz (1f) oder Öffnungen aufweist, die über eine Breite des Innenraums (HV) verteilt sind; und
- sich unter einem Sitzkissenteil der Rücksitze und/oder vollständig unter der Ebene von Fensteröffnungen (W) befindet, die mit Scheiben verschlossen sind.

9. System nach einem der vorhergehenden Ansprüche, wobei die Mittel (22) zur Diffusion von gereinigter Luft über ein oder mehrere Diffusionsorgane (17) für die Diffusion von mindestens einem flüchtigen, trockenen Produkt verfügen, das aus einer bioziden Komponente und einem Duftstoff ausgewählt ist, wobei jedes der Diffusionsorgane (17) in fluidischer Verbindung mit einem entsprechenden Produktbehälter steht, wobei die Diffusionsorgane (17) vorzugsweise von den Verkleidungen (24a, 24b) getragen werden oder fest mit ihnen verbunden sind.

10. Kombinierte Verwendung einer Filteranordnung (2) und von Mitteln (22) zur Diffusion von gereinigter Luft in einem Innenraum (HV) in einem Fahrzeug (V) für Passagiere und/oder einen Fahrer, **dadurch gekennzeichnet, dass** die Filteranordnung (2) und die Mittel (22) zur Diffusion von gereinigter Luft am Fahrzeugheck miteinander verbunden sind, um das Luftreinigungssystem (1) nach einem der vorhergehenden Ansprüche zu bilden, wodurch die gereinigte Luft, die von den Mitteln (22) zur Diffusion von Luft in verschiedenen Positionen neben dem Dach (9) des Fahrzeugs (V) in der von vorne nach hinten verlaufenden Richtung des Fahrzeugs durch die Verkleidungen (24a, 24b) hindurch verteilt wird, frei von Feinstaubpartikeln PM0,3 und PM0,1 ist.

11. Verwendung nach Anspruch 10, wobei sich alle Filterkomponenten und aktiven Komponenten zur Reinigung und Zirkulation der Luft des Systems (1) in einem hinteren Teil (42) des Fahrzeugs (V) befinden, wodurch ein Betrieb des Systems unabhängig von dem Betrieb einer Klimaanlage (20) ermöglichen, die im vorderen Bereich des Fahrzeugs (V) integriert und so eingerichtet ist, dass sie die Klimatisierung und die Temperaturregelung durch Filterung und Konditionierung einer von außerhalb des Innenraums (HV) entnommenen Luft realisiert.

12. Verwendung nach Anspruch 10 oder 11, wobei die Mittel (22) zur Diffusion von gereinigter Luft mindestens zwei Diffusionsbereiche umfasst, die voneinander durch eine Weichenvorrichtung (36) getrennt sind, die mindestens ein einstellbares Ventil aufweist, um einen Luftdiffusionsdurchsatz selektiv in Abhängigkeit von den Diffusionsbereichen einzustellen, wobei vorzugsweise einer der beiden Diffusionsbereiche so angeordnet ist, dass er einen vorderen Diffusionsbereich bildet, der einer ersten Sitzreihe des Fahrzeugs zugeordnet ist, und ein anderer der beiden Diffusionsbereiche so angeordnet ist, dass er einen hinteren Diffusionsbereich bildet, der einer zweiten Sitzreihe des Fahrzeugs zugeordnet ist.

## Claims

1. System (1) for purifying the air of a vehicle interior (HV) for passengers and/or the driver, in particular of an automobile interior, the system comprising:
- a filtering arrangement (2) including an air purifying filter medium (3) separating out at least the fine particle classes PM0.3 and PM0.1, said filter medium (3) being preferably class H13, and at least one layer (4) for capturing volatile compounds, in particular by molecular adsorption;
- a filtration chamber (5) wherein the air purifying filter medium (3) extends such that the filtering arrangement (2) delimits an upstream zone (Z1) in communication with one or more admission passages (6) of the system (1);
- an air circulation means (8) for circulating the air (F) coming from the vehicle interior (HV) in an air treatment part (10) including the filtering arrangement (2) and a duct (7) for discharging air purified by the filtering arrangement (2), knowing that the air circulation means (8) makes it possible to expel, in the discharge duct (7), the purified air (F') circulating in a downstream zone (Z2) of the filtration chamber (5);
- a prefilter (11) for capturing solid and/or liquid particles upstream of the filter medium (3) in an air circulation direction selectively permitted by the air circulation means (8);
wherein the discharge duct (7) is in communication with at least one branch, preferably two branches (7a, 7b), the duct and/or each branch forming an ascending pipe in communication with purified air diffusion means (22) that extend, from a high end (7c) of the branch, towards the front of the automobile interior (HV),
and wherein the purified air diffusion means (22) comprise at least one ducting (24a, 24b), extending along the roof (9) of the vehicle interior (HV) so as to diffuse the purified air downwards into the vehicle interior, each ducting (24a, 24b) having perforations and/or air-permeable zones (23a) which are provided both in the proximal position (P1) of a rear end (9a) of the roof and in a distal position (P2), at which said ducting (24a, 24b) covers all or part of a front end (9b) of the roof from the inside.

2. System according to claim 1, wherein the purified air diffusion means (22) delimit peripheral air circulation passages (23) with respect to a passenger occupancy zone in the vehicle interior,
and wherein the filtration chamber (5), the discharge duct (7) and the peripheral air circulation passages are part of the same independent and separate air circulation circuit of the conditioning and temperature control equipment (20) by filtering and conditioning air taken from outside the vehicle interior, the air circulation means (8) being configured to be placed in a rear region of the vehicle interior with reference to a so-called forward direction of circulation of the vehicle (V) defining the vehicle interior (HV).

3. System according to claim 1 or 2, wherein the perforations and/or air-permeable zones (23a), for each ducting (24a, 24b), are integrated into a thickness (e) of ducting material, preferably without forming reliefs protruding on the outer surface of the ducting.

4. System according to any one of the preceding claims, wherein the air treatment part (10) comprises a housing (50) that delimits the filtration chamber (5) and is provided with an inlet (51), forming all or part of the admission passages (6), to circulate air admitted into the upstream zone (Z1) of the filtration chamber, the downstream zone (Z2) being:
- delimited by a pre-assembled filter element including the air purifying filter medium (3); and
- in communication with the discharge duct (7) via a low ventilation zone, vertically distal from the roof, equipped with at least one fan element which constitutes all or part of the air circulation means (8).

5. System according to any one of the preceding claims, wherein two sections of textile ducting (24a, 24b) are provided which are continuous, forming all or part of the at least one ducting, and which each extend continuously from the rear end (9a) of the roof to the front end (9b) of the roof each defining a useful section for diffusing purified air, preferably greater than a threshold, for example with a useful diffusion section at least equal to 4 dm².

6. System according to any one of the preceding claims, wherein the air purifying filter medium (3) extends annularly about a central axis (X) and is designed to make centripetal air filtration possible, the layer (4) for capturing volatile compounds being arranged internally with respect to the filter medium (3), extending around a hollow space (24), the hollow space (24) preferably forming a part of the downstream zone (Z2) of the filtration chamber (5).

7. System according to any one of the preceding claims, wherein the purified air diffusion means (22) selectively comprise flexible ducting, the ducting including a textile material (T1, T2) defining an outer coating of the ducting (24a, 24b), visible from the inside of the vehicle interior (HV) or covered by a protective layer more rigid than the ducting.

8. System according to any one of the preceding claims, wherein the prefilter (11) is configured to separate particles with a submillimetre characteristic diameter or size, the prefilter (11) being disposed at an inlet of the system which:
- has at least one elongated slot (1f) or openings distributed over a width of the vehicle interior (HV); and
- is located under a part of the rear seat cushions and/or completely below the level of window (W) openings blocked by windows.

9. System according to any one of the preceding claims, wherein the purified air diffusion means (22) have one or more diffusion members (17) for diffusing at least one volatile, dry product, selected from a biocidal component and a perfumed fragrance, each of the diffusion members (17) being in fluid connection with a corresponding product reservoir, the diffusion members (17) preferably being supported by or secured to the ducting (24a, 24b).

10. Combined use of a filtering arrangement (2) and means (22) for diffusing purified air in a vehicle interior (HV) for passengers and/or the driver, in a vehicle (V), **characterised in that** the filtering arrangement (2) and the means (22) for diffusing purified air are interconnected at the rear of the vehicle to form the air purification system (1) according to any one of the preceding claims, thanks to which the purified air diffused through ducting (24a, 24b) of the means (22) for diffusing air, in various positions adjacent to the roof (9) of the vehicle (V) according to the forward and rearward direction of the vehicle, is free of fine particles PM0.3 and PMO.1.

11. Use according to claim 10, wherein all of the filtration components and active components for purifying and circulating the air of the system (1) are located in a rear part (42) of the vehicle (V) making it possible for the system to operate independently of the operation of air conditioning equipment (20) integrated in the front of the vehicle (V) which is configured to perform air conditioning and temperature control by filtering and conditioning air taken from outside the vehicle interior (HV).

12. Use according to claim 10 or 11, wherein the purified air diffusion means (22) comprise at least two diffusion zones separated from one another by a switching device (36) having at least one adjustable valve, in order to adjust an air diffusion flow rate selectively depending on said diffusion zones, with preferably one of the two diffusion zones arranged to form a front diffusion zone, associated with a first seat row of the vehicle and another of the two diffusion zones arranged to form a rear diffusion zone, associated with a second seat row of the vehicle.
